# EUROPEAN PATENT APPLICATION

(11) **EP 4 265 722 A2**
(43) Date of publication of application: **25.10.2023**
(21) Application number: 21865322.8
(22) Date of filing: 20.12.2021
(51) Int. Cl.: C12N 15/10, C12N 15/113

(54) **PROCESS FOR CONSTRUCTION OF A SINGLE VECTOR COMPRISING CAS9 AND SGRNA FOR MYCOBACTERIAL GENOME MODIFICATIONS**

(30) Priority: 18.12.2020 BR 102020026128
(71) Applicant: INSTITUTO BUTANTAN, 05503900 São Paulo - SP (BR)
(72) Inventor: LEITE, Luciana Cezar De Cerqueira, 05083-030 São Paulo - SP (BR); KANNO, Alex Issamu, 05359-001 São Paulo - SP (BR); MORAES, Luana, 05586-001 São Paulo - SP (BR)
(74) Representative: Antonucci, Alessandra
(86) International application number: PCT/BR2021/050560
(87) International publication number: WO 2022/126219

(57) **Abstract**

The present invention relates to a process for constructing a single vector comprising *cas9* and sgRNA ("all-in-one" vector) for mycobacterial genome modifications, comprising the steps of (a) providing the expression cassette of cas9 and the tracrRNA expression cassette with codons optimized for expression in mycobacteria and clone into a cloning vector; (b) sequentially cloning the sequences optimized in step (a) into a mycobacterial bridge vector; (c) selecting targeting sequences (crRNAs) specific to the gene of interest; (d) inserting the crRNAs selected in step (c) into the mycobacterial expression vector, upstream of the tracrRNA sequence, at position +1 downstream of an inducible promoter for the construction of the sgRNA cassette; and (e) adding at the end of each expression cassette a transcriptional terminator.

## Description

### Field of Application:

The present invention is part of the field of genetic engineering, more specifically, in the area of molecular biology and cell biology, since the present invention relates to a process for modifying the genome of mycobacteria through a single vector using the CRISPR-Cas system.

### Fundamentals of the Invention and State of the Art:

*Bacillus Calmette-Guérin* (BCG) is a live attenuated strain of *Mycobacterium bovis* and the only licensed vaccine against *tuberculosis* (TB). It was developed a century ago and is still in use today. BCG is usually given at birth and is very effective in protecting children against severe forms of *tuberculosis.* Over time, protection diminishes, and epidemiological evidence suggests variable efficacy in adults against the pulmonary form of the disease, ranging from 0 to 80%. Therefore, there are many strategies being investigated for the development of new and better vaccines.

Recombinant BCG (rBCG) is an attractive strategy to produce improved vaccines against *tuberculosis.* Since the development of mycobacterial expression vectors, many rBCG strains have been generated expressing a variety of antigens, cytolysins, and cytokines.

The CRISPR/*Cas9* system is a promising option to boost the generation of rBCG strains. This recently developed system has been used to manipulate the genome of various organisms. The technique has become a system wherein a simple guide RNA design allows reaching any sequence of interest, with the only condition of being adjacent to a PAM domain (NGG). Through this technique it is possible to easily inactivate or delete genes (knockout), modify or insert genes (knockin) or silence genes (knockdown), among other modifications.

Some prior art documents describe the application of CRISPR/Cas9 technology in mycobacteria.

Chinese patent application CN 110791468 A, published on February 14, 2020, on behalf of JIANGNAN UNIVERSITY, entitled: "CONSTRUCTION METHOD AND APPLICATION OF MYCOBACTERIA GENETIC ENGINEERING BACTERIA" describes a method of constructing genetically modified mycobacteria through the CRISPR/Cas9 system, wherein an initial vector (pMV261) is used to clone the *Cas9* protein expression cassette for mycobacteria and a second editing vector (pMVC) for inactivation (knockout) of the *17β-hsd* gene of a mycobacteria.

Chinese patent application CN 110066829 A, published on July 30, 2019, also in the name of JIANGNAN UNIVERSITY, entitled: "CRISPRICAS9 GENE EDITING SYSTEM AND APPLICATION THEREOF" describes a gene editing system through the technology CRISPR/Cas9, wherein the system uses two plasmids pML-*Cas9* and pJM-sgRNA for inactivation (knockout) of the *ksdd* gene of a mycobacteria, such as *Mycobacterium aureus.*

Differently, the present invention proposes a simplified process for mycobacterial genome modifications, wherein, in an innovative and surprising way, there is proposed a single "all-in-one" vector that comprises all the elements necessary for the knockout of genes through controlled expression of *Cas9* and sgRNA in mycobacteria.

The use of a single "all-in-one" vector to carry out controlled expression of *Cas9* and sgRNA in a single step is already described in some prior art documents.

International application No. WO 2019/213776 A1, published on November 14, 2019, on behalf of UNIVERSITÉ LAVAL, entitled: "CRISPR/CAS9 SYSTEM AND USES THEREOF" describes methods and products for modifying nucleic acids using a CRISPR/Cas9 system. In one embodiment, said document discloses the construction of an all-in-one hepatotropic rAAV (recombinant adeno-associated virus) vector to deliver holo-St1 *Cas9* (St1 *Cas9* + sgRNA) to the liver of newborn mice. This is aimed at altering genes in the cells of mice. In contrast, the process of the present invention involves a mycobacterial vector (non-rAAV) that allows deletions and insertions of genes in mycobacteria (not in mouse liver) in a single step, and specifically inactivates the *lysA* gene using CRISPR/Cas9 to generate an unlabeled auxotrophic BCG strain.

The article titled "A CRISPR-CAS9 ASSISTED NON-HOMOLOGOUS END-JOINING STRATEGY FOR ONE-STEP ENGINEERING OF BACTERIAL GENOME", published on November 24, 2016 in SCIENTIFIC REPORTS, on behalf of Tianyuan Su *et al.,* discloses that CRISPR-*Cas9* technology aided the non-homologous end joining strategy (CA-NHEJ) for the rapid and efficient inactivation of *Escherichia coli* gene(s) in a manner independent of homologous recombination and without the use of selective marker. This process involves two steps of bacterial transformation and requires the presence of other nucleotide sequences (Ku and LigD) in the *Cas9* expression vector. In contrast, the process of the present invention allows deletions and insertions of genes in mycobacteria in a single step (with a single vector), and specifically inactivates the *lysA* gene using CRISPR/*Cas9* to generate an unlabeled auxotrophic BCG strain. Transformation and expression in mycobacteria are a much less efficient process and requires vectors with an origin of replication in mycobacteria.

North American application US 2018/010151 A1, published on January 11, 2018, on behalf of DSM IP ASSETS BV entitled: "A CRISPR-CAS SYSTEM FOR A YEAST HOST CELL" describes a CRISPR-Cas system into a single vector for yeast modification. In one embodiment, an all-in-one yeast expression vector was constructed containing *CAS9*, guide RNA, and an antibiotic resistance marker. In contrast, the process of the present invention allows deletions and insertions of genes in mycobacteria in a single step, and specifically inactivates the *lysA* gene using CRISPR/*Cas9* to generate an unlabeled auxotrophic BCG strain. Transformation and expression in mycobacteria is a much less efficient process and requires vectors with an origin of replication in mycobacteria. Unlike the present invention, US patent application 2018/010151 A1 uses two constitutive promoters (TEF1 and SNR52) for expression of *Cas9* and the guide.

Therefore, it is notable that a process for specifically mycobacterial genome modifications via a single "all-in-one" vector comprising all elements necessary for gene knockout through controlled expression of *Cas9* and sgRNA is new and inventive in view of the state of the art.

It is important to emphasize that the option of simplifying the process of mycobacterial genome modifications is important because the cultivation of mycobacteria requires a much longer time than for *E. coli* or yeast (~1 month compared to 1-2 days) and it is important to limit the number of subcultures for the viability of the bacterium.

In addition, the use of two or more vectors includes the use of selection markers such as those that confer resistance to antibiotics. Additionally, it is known that the expression of *Cas9* can be toxic to the host cell, and furthermore, it differs in the constitution of the promoters. In addition, the inducible expression is positive because it better controls these factors (toxicity and off-targets).

Therefore, in an embodiment of the process proposed by the present invention, it is possible to construct a complete vector containing CRISPR/*Cas9* to functionally inactivate (knockout) the expression of the *lysA* gene of mycobacterial strains, and additionally it was possible to demonstrate the efficient complementation of these knockouts with extrachromosomal vectors containing *lysA* also expressing the adjuvant *LTAK63.*

These results demonstrate the utility of CRISPR/*Cas9* in efficiently mutating at specific positions in the mycobacterial genome. The mutations proved to be irreversible, further reinforcing the use of CRISPR/*Cas9* to generate new and improved TB vaccines.

Complementation showed high plasmid stability; together with the expression of *LysA,* these strains were also able to stably express the adjuvant *LTAK63.* The results suggest that the complemented auxotrophic rBCG strains generated by the present invention would have the necessary properties to induce improved protection against *M. tuberculosis* challenge.

Therefore, no prior art documents describe or suggest a CRISPR-Cas process for mycobacterial genome modifications through a single vector, preferably for the inactivation of the *lysA* gene using CRISPR/Cas9 to generate an unmarked auxotrophic BCG strain.

### Summary of the Invention:

The present invention will provide significant advantages in the field of genetic engineering.

In a first aspect, the present invention relates to a process for constructing a single vector comprising *cas9* and sgRNA ("all-in-one" vector) for mycobacterial genome modifications.

### Brief description of the invention:

The structure and operation of the present invention, along with additional advantages thereof, can be better understood by referring to the attached images and the following description.
Figures 1A-C refer to a schematic representation of the vectors for CRISPR gene editing and auxotrophic complementation, where A) is the pKLM-CRISPR-*lysA* (x) vector used for genome editing in BCG and *M. smegmatis* and B) and C) are pAN71*-LTAK63-lysA*(t) and pAN71*-LTAK63-lysA*(c) vectors used to complement auxotrophic strains by disrupting the *lysA* gene.
Figures 2A-B represent images of tetracycline-inducible expression of *Cas9* in: A) BCG and B) *M. smegmatis.*
Figures 3A-B represent images of the phenotypic screening of BCGΔ*lysA* and *M. smegmatis*Δ*lysA,* wherein colonies of A) BCG and B) *M. smegmatis* were then seeded onto mirror plates with and without lysine.
Figures 4A and 4C represent the characterization of CRISPR/*Cas9*-induced *lysA* mutations in A) BCG and C) *M. smegmatis,* and Figures 4B and 4D represent that mutations generated by CRISPR/*Cas9* which resulted in interrupted translation of lysine, regardless of the amount of nucleotides deleted.
Figure 5 represents images of the serial passage of BCGΔ*lysA* that does not revert to the wild-type phenotype.
Figures 6A-B plot the growth curves of complemented auxotrophic strains compared to wild-type strains of A) BCG and B) *M. smegmatis.*
Figures 7A-B represent images of *LTAK63* expression in auxotrophic BCG and *M. smegmatis* complemented by A) BCG and B) *M. smegmatis.*
Figure 8 represents the alignment of the sequences of the *M. bovis* and *M. smegmatis lysA* genes by GeneDoc.
Figures 9 graphically represent different levels of *Cas9* expression depending on tetracycline concentration and induction time.
Figure 10 plots the differences in band sizes observed by PCR after genome editing.
Figure 11 presents images of the cure of auxotrophic BCG clones transformed with pKLM-CRISPR-lysA in the first pass.

### Detailed description of the invention:

Although the present invention may be susceptible to different embodiments, the drawings and the following detailed discussion show a preferred embodiment with the understanding that the present embodiment is to be considered an exemplification of the principles of the invention and is not intended to limit the present invention to which has been illustrated and described in this report.

The present invention relates to a process for constructing a single vector comprising *cas9* and sgRNA ("all-in-one" vector) for mycobacterial genome modifications.

This process comprises the following steps:
a) Build and provide the *cas9* expression cassette and the *tracrRNA* expression cassette with codons optimized for expression in mycobacteria and clone into the pUC57 vector, or any other cloning vector;
b) Sequentially clone the sequences optimized in step (a) into a mycobacterial bridge vector consisting of any mycobacterial expression vector that comprises a mycobacterial origin of replication and an antibiotic resistance marker;
c) Select targeting sequences (crRNAs) specific to the gene of interest;
d) Insert the crRNAs selected in step (c) into the mycobacterial bridge vector, upstream of the *tracrRNA* sequence, at position +1 downstream of an inducible promoter for the construction of the sgRNA cassette; and
e) Add a transcription terminator at the end of each expression cassette.

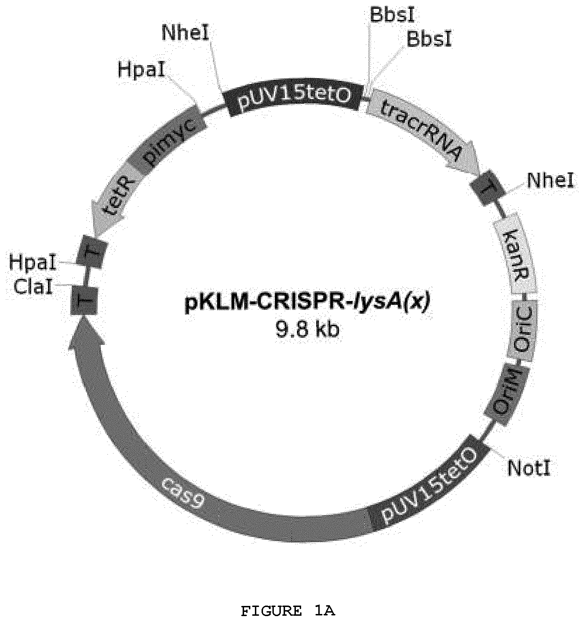

Figure 1A presents an embodiment of the construction of the "all-in-one" vector obtained through the process described here.

Thus, this process allows obtaining an "all-in-one" vector for mycobacterial genome modifications such as gene inactivation or deletion (knockout), gene modification or insertion (knockin) or gene silencing (knockdown).

Preferably, the mycobacteria are selected from the group consisting of the *tuberculosis* complex (*Mycobacterium tuberculosis* (MTB), *Mycobacterium bovis* (BTB), *Bacillus Calmette-Guérin* (BCG) and *Mycobacterium smegmatis*).

Said mycobacterial bridge vector comprises an antibiotic resistance marker such as kanamycin (kanR); origin of replication in *E. coli* (oriC) and origin of replication in mycobacteria (oriM). There can be used any vector which contains these components. In one embodiment, the pJH152 vector is used, but not limited to, and any mycobacterial expression vector containing a mycobacterial origin of replication and an antibiotic resistance marker may be used.

In step (a), to obtain the *cas9* expression cassette, reverse and forward primers complementary to the *cas9* sequence are used in PCR reactions. In one embodiment of the invention, forward SEQ ID NO:1 and reverse SEQ ID NO:2 primers are used to obtain the *cas9* expression cassette in PCR reactions.

To obtain the "all-in-one" vector, any inducible system is further used, preferably the tetR/tetracycline inducible system, wherein in step (a) the regulatory cassette of the inducible system is further constructed, preferably the cassette Tet regulator (*TetR*), capable of expressing a codon-optimized *Cas9* in mycobacteria.

It is worth noting that step (a) is performed by the synthesis of genes with optimized codons for expression in mycobacteria, wherein the optimization of the genes is performed in a synthetic way, where the nucleotides of the genes to be optimized are modified following the table of codons that are preferentially used by the microorganism to compose the amino acid. Said codon table is common knowledge in molecular biology.

Thus, still in step (a), any inducible system is used, preferably the *tetR*/tetracycline inducible system, wherein in step (a) the regulatory cassette of the inducible system is further synthesized, preferably the Tet regulatory cassette (*TetR*), wherein forward and reverse primers complementary to the *TetR* sequence are used in PCR reactions. In one embodiment of the invention, to obtain the Tet regulatory cassette (*TetR*) forward SEQ ID NO:3 and reverse SEQ ID NO:4 primers are used in PCR reactions.

Still in step (a), to obtain the *tracrRNA* expression cassette, forward and reverse primers complementary to the *tracrRNA* sequence are used in PCR reactions. In one embodiment of the invention, forward SEQ ID NO:5 and reverse SEQ ID NO:6 primers are used to obtain the *tracrRNA* expression cassette in PCR reactions.

It is important to emphasize that the use of codon-optimized *cas9* (by DNA sequence synthesis) is essential for the construction of the "all-in-one" vector of the present invention. Synthesis is the process of modifying one or more nucleotide sequences in order to improve the expression of said protein in the organism of interest. This change modifies the native codons for the preferred codons of the host organism. In practice, an *in silico* analysis is performed and the new DNA sequence is chemically synthesized.

By using a tetracycline-inducible system, it was possible to characterize the expression of a codon-optimized *Cas9* in mycobacteria, such as in *M. smegmatis* and BCG, peaking at 8h and 24h of induction, respectively (they were tested from 4 to 120h), at a concentration of up to 200 ng/mL (concentrations from 20 to 2000 ng/mL were tested). Higher concentrations of tetracycline lead to a decrease in viability.

It is important to emphasize that any inducible system can be used in place of *tetR*/tetracycline.

Tetracycline hydrochloride was used for the optimization in step (a) instead of anhydrotetracycline in order to use a more commercially accessible reagent, determined as *Cas9* expression at the same concentration previously characterized by others using tetracycline-inducible systems.

In step (b), the cassette of the inducible system used, preferably the *tetR* cassette, is constitutively expressed by the pimyc promoter, or any other constitutive promoter active in mycobacteria.

In one embodiment of the invention, in step (c) specific crRNAs are selected for the construction of sgRNAs to target the gene of interest in mycobacteria.

In an exemplary embodiment of the invention, for purposes of clarity, forward SEQ ID NO:7 and reverse SEQ ID NO:8 primers are used in PCR reactions to obtain the *lysA* gene sequence.

In an exemplary embodiment of the invention, in step (c) the specific crRNAs for the construction of sgRNAs to target the *lysA* gene in mycobacteria are obtained through the following probes:
- cRNA*lys*A20-BCG probe - shows forward SEQ ID NO:9 and reverse SEQ ID NO:10 primers in PCR reactions to obtain a specific sequence for sgRNA to target the *lysA* gene in BCG;
- cRNA*lysA*88-BCG probe - presents the forward SEQ ID NO:11 and reverse SEQ ID NO:12 primers in PCR reactions to obtain a specific sequence for sgRNA to target the *lysA* gene in BCG;
- cRNA*lysA*394-smeg probe - displays forward SEQ ID NO:13 and reverse SEQ ID NO:14 primers in PCR reactions to obtain a specific sequence for sgRNA to target the *lysA* gene in *M. smegmatis;*
- cRNA*lysA*820-smeg probe - displays forward SEQ ID NO:15 and reverse SEQ ID NO:16 primers in PCR reactions to obtain a specific sequence for sgRNA to target the *lysA* gene in *M. smegmatis;* and
- cRNA*lysA*123-smeg probe - displays forward SEQ ID NO:17 and reverse SEQ ID NO:18 primers in PCR reactions to obtain a specific sequence for sgRNA to target the *lysA* gene in *M. smegmatis.*

In step (d), it is important to note that the expression of *Cas9* and sgRNA is controlled by an inducible promoter, preferably by a tetracycline-inducible promoter. In one embodiment of the invention, said tetracycline-inducible promoter is the pUV15tetO promoter.

In step (e), transcriptional terminators (T) are introduced after each expression cassette. In addition, restriction sites used for cloning are also indicated and two restriction sites, preferably Bbs I, are introduced for easier cloning of the crRNA upstream of the *tracrRNA.*

Therefore, through the process described herein, an "all-in-one" vector containing all the necessary elements for mycobacterial genome modifications is obtained.

In one embodiment of the invention, the process described herein constructs a vector of SEQ ID NO:19 that applies the CRISPR/*Cas9* system to functionally inactivate (knockout) *LysA* expression of mycobacterial strains.

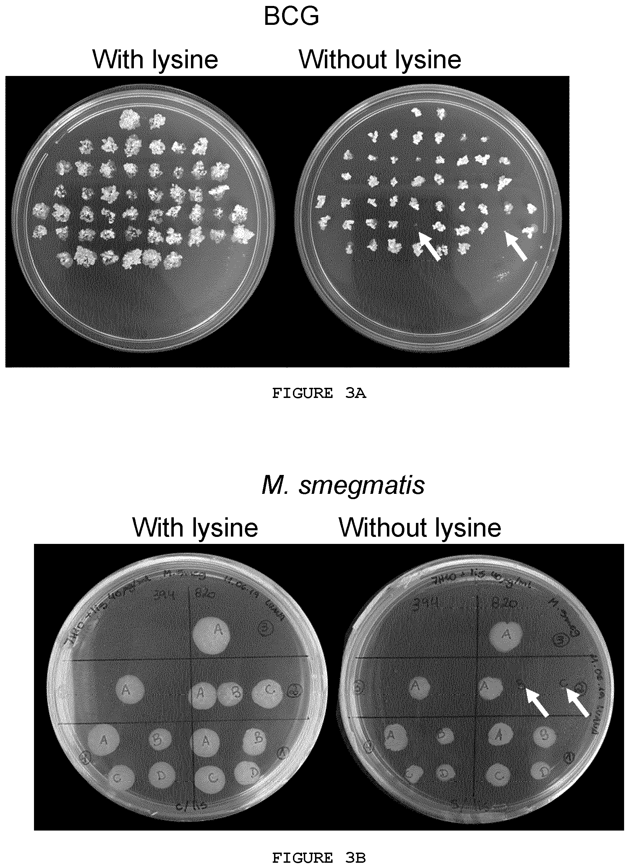

To prove such embodiment, mycobacterial strains are grown on solid medium supplemented with lysine, but not on medium without lysine supplementation (Figure 3).

To prove such embodiment, these knockouts were efficiently complemented with extrachromosomal vectors containing *lysA* and also expressing the adjuvant *LTAK63.*

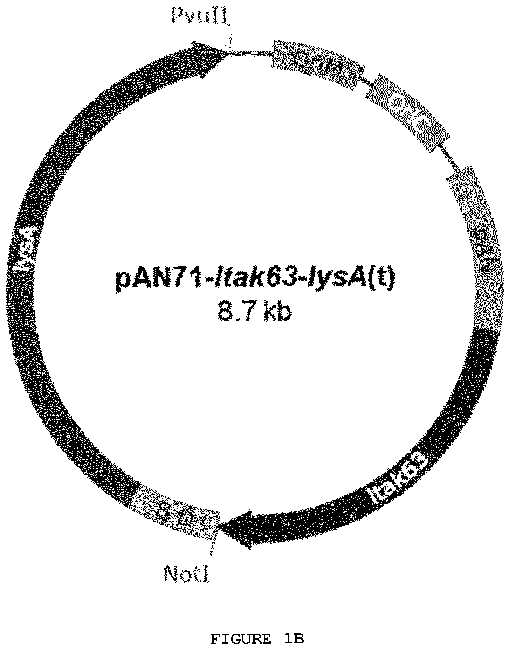

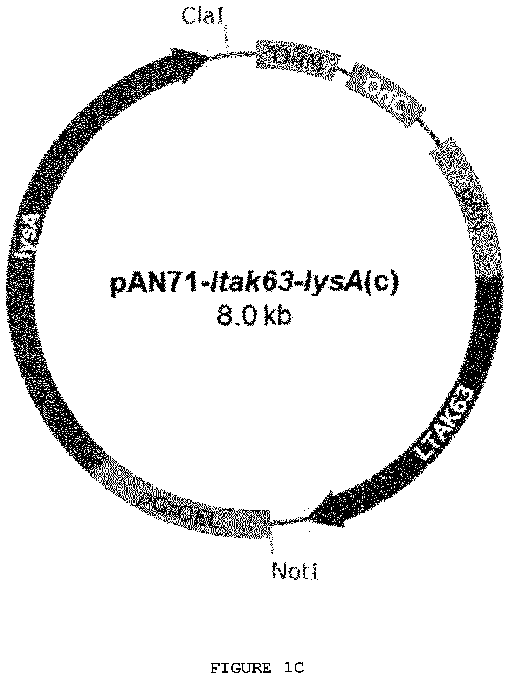

In this sense, two different constructs were evaluated using *LysA* complementation. The pAN71-*LTAK63* vector contains the PAN promoter, which directs lower expression of the *LTAK63* antigen. This vector was used to clone the *lysA* gene in tandem with *LTAK63,* using the same PAN promoter, or to clone a *lysA* expression cassette with the PGrOEL promoter, thus generating the pAN71-*LTAK63-lysA*(t) vectors (Figure 1B) of SEQ ID NO:20 and the pAN71-*LTAK63-lysA(c)* vector (Figure 1C) of SEQ ID NO:21.

The *pAN71-LTAK63-lysA(t)* vector also has a Shine-Dalgarno (SD) sequence between the *LTAK63* and *lysA* genes. Also, they both contain an OriC and an OriM. The kanamycin resistance marker is disrupted by restriction digestion using Cla I for *pAN71-LTAK63-lysA(t)* and Nsi I for pAN71-*LTAK63-lysA(c)*. Complementation showed high plasmid stability; together with *LysA* expression, wherein these strains were also able to stably express the adjuvant *LTAK63.*

Therefore, surprisingly, the process described here allows obtaining an "all-in-one" vector for mycobacterial genome modifications and, therefore, for obtaining auxotrophic recombinant strains complemented with properties necessary to induce an improved protection against the *M. tuberculosis* challenge.

### Preferred Embodiments of the Invention:

### - Strains, media and growth conditions:

*E. coli* DH5α strain, *M. smegmatis* mc² 155 strain, *M. bovis* BCG Danish strain and their derivatives were used in one embodiment of the present invention. *E. coli* was used for the cloning steps and was grown in LB medium (Luria Bertani). The *M. smegmatis* strain was grown on Middlebrook 7H9 (Difco) supplemented with 0.5% glycerol (Sigma) and 0.05% Tween 80 (Sigma) (MB7H9) or seeded on Middlebrook 7H10 agar supplemented with 0.5% of glycerol (MB7H10). The *M. bovis* BCG strain was grown in Middlebrook 7H9 supplemented with 10% OADC (Becton Dickinson), 0.5% glycerol and 0.05% Tween 80 (MB7H9-OADC) or seeded on 7H10 agar supplemented with 0.5% glycerol and OADC (MB7H10-OADC). *E. coli* and *M. smegmatis* were cultivated at 37°C. BCG was cultivated at 37°C and 5% CO₂. When indicated, kanamycin (20 pg/ml), tetracycline (Tc) (200 ng/ml) and/or lysine (40 pg/ml) were also used.

### - Preparation and transformation of competent cells:

*E. coli* DH5α was grown in 400 ml of LB to OD600 0.4-0.6 when cells were washed twice with ice-cold 0.1 M CaCl₂. Competent cells were resuspended in 10% glycerol and stored in 100 µL aliquots at -80°C until use. *E. coli* transformation was performed by heat shock. For the preparation of competent *M. smegmatis,* a single colony was used to inoculate 50 mL of MB7H9 which was incubated at 37°C under 200 rpm in an orbital shaker (Gyromax 737R, Amerex, Lafayette, CA, USA) until OD600 0.6-0.8. The culture was kept on ice for 90 min and then washed twice with ice-cold 10% glycerol. The culture was resuspended in 1 mL of 10% glycerol and 100 µL aliquots stored at -80°C until use. For transformation, cells were defrosted on ice and incubated for 10 min with 300-500 ng of plasmid DNA. Electroporation was performed using the Gene Pulser II device (BioRad, Hercules, CA, UK) configured for 2.5 Kv, 25 µF and 200 Ω. Cells were resuspended in 2 mL of MB7H9 and incubated at 37°C for 2h and subsequently seeded in MB7H10 containing kanamycin and incubated at 37°C for 5-7 days until visible colonies appeared.

BCG was cultured in 50 ml of MB7H9-OADC and incubated at 37°C for 1-2 weeks until OD₆₀₀ 0.6-0.8. As before, cells were washed twice with ice-cold 10% glycerol, resuspended in 1 mL of 10% glycerol and 100 µL aliquots stored at -80°C until use. After electroporation (2.5 Kv, 25 µF and 1000 Ω), the cells were resuspended in 1 mL of MB7H9-OADC and incubated at 37°C, 5% CO₂ for 16h. Then, cells were seeded on MB7H10-OADC plates and incubated as before until visible colonies appeared (2-3 weeks). For the preparation of competent auxotrophic strains, lysine was added to the medium.

### - Construction of the all-in-one vector containing cas9 and sgRNA for lysA disruption:

The pJH152 vector (provided by Dr. Stewart Cole, EPFL, Lausanne, France) was used to sequentially clone the *cas9* expression cassette (in this case, at the Not I and Cla I sites, with these sites also synthesized at the ends of the optimized expression cassettes), the Tet regulator cassette (tetR) and the *tracrRNA* sequence (*Cas9* nuclease recruitment sequence) at the Hpa I and Nhe I positions as shown in Figure 1. Other restriction sites can be used at this or in other expression vectors.

The crRNA selection was made using the Cas-Designer tool on the R-GENOME website (*http:*//*www.rgenome.net*/*cas-designer*/). The selected specific targeting sequences, crRNAs, were inserted at the Bbs1 positions of the "all-in-one" vector, henceforth pKLM-CRISPR-*lysA* (x), upstream of *tracrRNA,* at position +1 downstream of the promoter, constituting the sgRNA cassette. The expression of *Cas9* and sgRNA is controlled by a tetracycline-inducible promoter (pUV15tetO). These sequences were codon-optimized for expression in mycobacteria (GenScript) and cloned into pUC57. At the end of each expression cassette, a transcriptional terminator (T4g32) was added. The orientation of the fragments was confirmed by sequencing. As already mentioned, the final vectors were named pKLM-CRISPR-*lysA*(x), shown in Figure 1A.

In addition, the plasmid contains the origin of replication for *E. coli* (OriC) and for mycobacteria (OriM), and a kanamycin resistance marker (kanR). The complete list of plasmids and oligonucleotides used in the present invention is shown in Table 1 and Table 2.

**Table 1 - Characteristics of the plasmids used in the present embodiment of the invention.**

| **Vector** | **Relevant features** | **References** |
|---|---|---|
| pJH152 | Kan^{R} (Tn10), *E. coli* origin of replication (pUC), mycobacterial origin of replication (ori-myco), pα-Ag promoter, MSC, MSP-1, and *lysA* gene | COLE, S.T. *et al* (2006) |
| pUC57 | *lacZ,* bla (Ap^{R}), MCS, rep (pMB1)-2710bp | GenScript |
| pUC57-cas9 | pUC57, containing codon-optimized *cas9* gene expression cassette | The present study |
| pUC57-tetR | pUC57, containing the *tetR* gene expression cassette | The present study |
| pUC57-tracrRNA | pUC57, containing the *tracrRNA* gene expression cassette | The present study |
| pKLM-CRISPR-*lysA* (x) | pJH152, containing *cas9, tetR* and *tracrRNA* gene expression cassettes | The present study |
| pLA71 | mycobacterial promoter (BlaF*), Kan^{R} -12000 bp | LIM, E. M. *et al.* (1995) |
| *pAN71-LTAK63* | pLA71, replacing the PAN promoter with PBlaF*, loading codon optimized from *LTAK63* | NASCIMENTO, I. P. *et al* (2017) |
| pAN71*-LTAK63-lysA(t)* | *pAN71-LTAK63,* carrying the *lysA* gene in tandem and the Kan^{R} gene deletion | The present study |
| pAN71-*LTAK63-lysA(c)* | *pAN71-LTAK63,* carrying the *lysA* gene on cassette and the Kan^{R} gene deletion | The present study |
| pCas | repA101(Ts) kan Pcas-*cas9* ParaB-Red *lacIq* Ptrc-*sgRNA*-pMB1 | Jiang *et al.* (2015) |

**Table 2 - Oligonucleotides used as primers in PCR reactions, in genome sequencing or as specific sgRNA - crRNA sequences).**

| SEQ ID NO | Probes | 5'-3' Sequences | PCR product |
|---|---|---|---|
| 1 / 2 | *Cas9* | TGGCATCCGTGGCGCGGCCG C AGAAATATTGGA/ TCGCTGGCATCGATAAAGGG GACCTCTAGGG | For *cas9* sequencing |
| | | | |
| 3 / 4 | *TetR* | TATCGATGCCAGCGAGTCAT GAGGTNAC/ TCTACGTA GTCGACGCATGCCTCGAG | For *tetR* sequencing |
| 5/ 6 | *tracrRNA* | GCGTCGAC TACGTAGAATTCAGAAATAT TGGATCGTCGG/ CTAGTTAACTACGTCGACAT TCTAGATTAATTAAAAAAAA GCACCGACTCGGT | For *tracrRNA* sequencing |
| 7 / 8 | *LysA* | TAGCAGCTG GCGGCCGCTCATTGACCCTT CACCTCCAGGCTCAG/ TAGCAGCTG GCGGCCGCTCATTGACCCTT CACCTCCAGGCTCAG | For *lysA* sequencing |
| 9 / 10 | crRNA-*lysA20-*BCG | GGGAGTGCAAACGTCCAGAC AGAG/ AAACCTCTGTCTGGACGTTT GCAC | Specific sequence of sgRNAs to cleave *lysA* in BCG |
| 11 / 12 | crRNA-*lysA88-*BCG | GGGAGTCGGAAAGCACTGCG AAAG/ AAACCTTTCGCAGTGCTTTC CGAC | Specific sequence of sgRNAs to cleave *lysA* in BCG |
| 13 / 14 | crRNA-*lysA394-*smeg | GGGATGCACCGTGATCCGCG AGGCCGG/ AAACCCGGCCTCGCGGATCA CGGTGCA | Specific sequence of sgRNAs to cleave *lysA* in *M. smegmatis* |
| | | | |
| 15 / 16 | crRNA-*lysA820-*smeg | GGGACAGATGTCCATCGTGG ATCTCGG/ AAACCCGAGATCCACGATGG ACATCTG | Specific sequence of sgRNAs to cleave *lysA* in *M. smegmatis* |
| 17 / 18 | crRNA-*lysA123-*smeg | GGGAGGTTCGCGGTGCCGAC GGGGTGG/ AAACCCACCCCGTCGGCACC GCGAACC | Specific sequence of sgRNAs to cleave *lysA* in *M. smegmatis* |

### - Construction of the complementation vector

The pAN71-LTAK63 vector contains the PAN promoter, which drives low expression of the *LTAK63* antigen. This vector was used to clone the *lysA* gene in tandem with *LTAK63* using the same P_{AN} promoter or to clone a *lysA* expression cassette with the P_{GrOEL} promoter, thus generating pAN71-*LTAK63-lysA(t)* (Figure 1B) and pAN71-*LTAK63- lysA*(c) (Figure 1C). pAN71-*LTAK63-lysA*(t) also has a Shine-Dalgarno sequence upstream of *lysA.* Also, they both contain an OriC and an OriM. The kanamycin resistance marker was disrupted by restriction digestion using Cla I for pAN71*-LTAK63-lysA*(t) and Nsi I for pAN71-*LTAK63-lysA*(c) (positions not shown in Figure 1, b).

Therefore, Figure 1 shows a schematic representation of the vectors for editing the *lysA* gene using the CRISPR technique and auxotrophic complementation. In Figure 1A, pKLM-CRISPR-*lysA* (x) vectors are used for genome editing in BCG and *M. smegmatis,* which contain the codon-optimized *cas9* gene expression cassette and a *tracrRNA* expression cassette, both under regulation of pUV15tetO, a tetracycline-inducible promoter. The tetR repressor is constitutively expressed by pimyc. Transcriptional terminators (T) were introduced after each expression cassette. Restriction sites used for cloning are also indicated. The two Bbs I sites were introduced for easier cloning of the crRNA upstream of the *tracrRNA.* The vectors also contain a kanamycin resistance marker (kanR); origin of replication in *E. coli* (oriC) and mycobacteria (oriM). Figures 1B-C are pAN71*-LTAK63-lysA*(t) and pAN71-*LTAK63-lysA*(c) vectors used for complementation of *lysA* auxotrophic strains. Both share the same features with oriM and oriC, the P_{AN} promoter and the *LTAK63* gene sequence. pAN71-*LTAK63-lysA*(t) expresses *lysA* in tandem with *LTAK63,* also including a Shine-Dalgarno (SD) sequence between the sequences. pAN71-*LTAK63-lysA*(c) expresses *lysA* under the control of the P_{GrOEL} promoter. Finally, the kanR site was disrupted after completion of the constructs, by restriction digestion and religation without the deleted fragment.

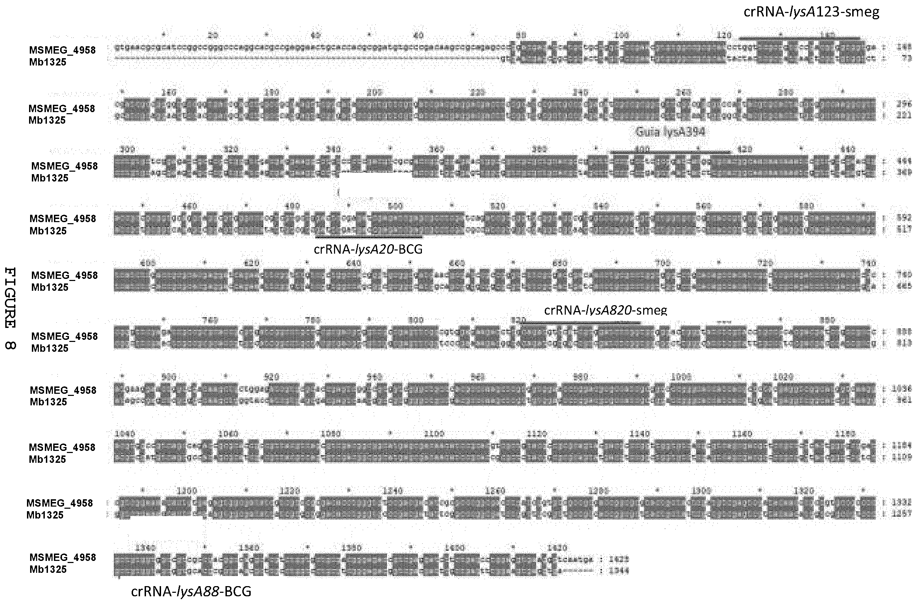

The positions of complementarity between crRNA and the mycobacterial genome (BCG and *M. smegmatis*) are represented in Figure 8.

### - Genome editing in M. bovis BCG and M. smegmatis.

The sgRNA selection was made using the Cas-Designer system on the R-GENOME website (*http:*//*www.rgenome.net*/*cas-designer*/)*.* After selection, three sgRNA sequences were chosen and their oligonucleotides hybridized and cloned into pKLM-CRISPR-*lysA*(x) at restriction positions Bbs I. After transformation of *M. smegmatis* and BCG with pKLM-CRISPR-*lysA*(x), kanamycin-resistant colonies were recovered and cultured in 5 mL of MB7H9 and MB7H9-OADC, respectively. These cultures were used as a pre-inoculum to obtain a fresh culture at OD₆₀₀ 0.1. After 2h or 24h of incubation, tetracycline was added, and the culture was maintained for 8h (*M. smegmatis*) and 48h (BCG) at 37°C in a CO₂ incubator. After induction, cells were recovered to perform Western blot to analyze *Cas9* expression using anti-*Cas9* monoclonal antibodies 7A9-3A3 (1:1,000) (Santa Cruz Biotechnology) and also seeded in MB7H10 and MB7H10-OADC supplemented with lysine until visible colonies appear. Single colonies were then transferred to fresh mirror plates containing or not lysine. Growth with lysine supplementation, but not on standard medium, indicated successful knockouts.

### - Characterization of knockout mutants:

After the generation of the auxotrophic strains of *M. smegmatis* and BCG, the knockouts were cured to eliminate the pKLM-CRISPR-*lysA*(x) plasmid by plating the transformants on mirror plates containing or not kanamycin. A clone demonstrating growth only on the kanamycin-free plate indicated that the plasmid was lost and competent cells were prepared. Mutant colonies of functional *LysA* knockouts for both strains were used as a template to PCR amplify the *lysA* region of the genome and sequence. To assess the possibility of reversion to wild-type phenotype during *in vitro* culture, BCG knockout strains were sequentially re-inoculated every week for 8 passages when cells were seeded in MB7H10-OADC containing or not lysine.

### - Complementation of auxotrophic strains:

Transformation by electroporation was performed similarly to wild-type strains. BCGΔ*lysA* and *M*. *smegmatis*ΔlysA clones were grown in MB7H9-OADC or MB7H9, respectively, supplemented with lysine. The bacteria from the clones were thoroughly washed and made competent as described above.

After transformation with pAN71*-LTAK63-lysA* (t) and pAN71*-LTAK63-lysA* (c), growth curves were generated with each colony in the respective media. The curves were compared with wild-type strains to determine whether the complemented auxotrophic strains would show altered growth. Additionally, at an OD₆₀₀ of 1.0, 20 µg of protein extracts from recombinant BCG and *M. smegmatis* were separated by SDS-PAGE, transferred to PVDF membranes and blocked with 5% skimmed milk powder at 4°C for 16h. Concomitant expression of the *LTAK63* antigen was assessed by Western blot using antiserum from mice previously immunized with rLTK63 (1:1000) and a horseradish peroxidase-conjugated anti-mouse IgG antibody (A6782, Sigma).

### Tests carried out:

### - Successful construction of the all-in-one vector for lysA disruption by CRISPR/Cas9 and the complementation vectors for LysA and LTAK63 expression:

Through the process proposed by the present invention, all vectors necessary for the fulfillment of the present invention were successfully constructed. The use of codon-optimized *cas9* was essential for this construction. Another important aspect of this vector is the expression of *cas9* and sgRNA, both under the control of the tetracycline-inducible promoter, pUV15tetO.

For complementation with *lysA*, two strategies were used: (a) the *lysA* gene placed next to the *LTAK63* gene, both under the control of the same promoter, P_{AN}, giving rise to the tandem construct, pAN-*LTAK63-lysA*(t); or (b) *lysA* was inserted as a cassette, with its own promoter, P_{GroEL}, giving rise to pAN-*LTAK63-lysA*(c)*.*

### - Inducible expression of Cas9 by mycobacteria:

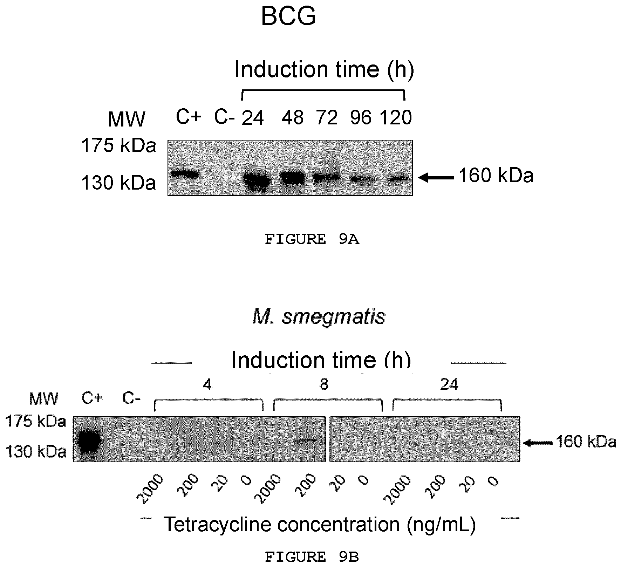

Initially, optimal conditions were established for the induction of *Cas9* with tetracycline (Figure 9).

Thus, Figure 9 graphically represents different levels of *Cas9* expression depending on tetracycline concentration and induction time, where molecular weight (MW) is shown; *E. coli* transformed with pKLM-CRISPR (C+); and the total extract of wild-type *M. bovis* BCG or wild-type *M. smegmatis* (C-).

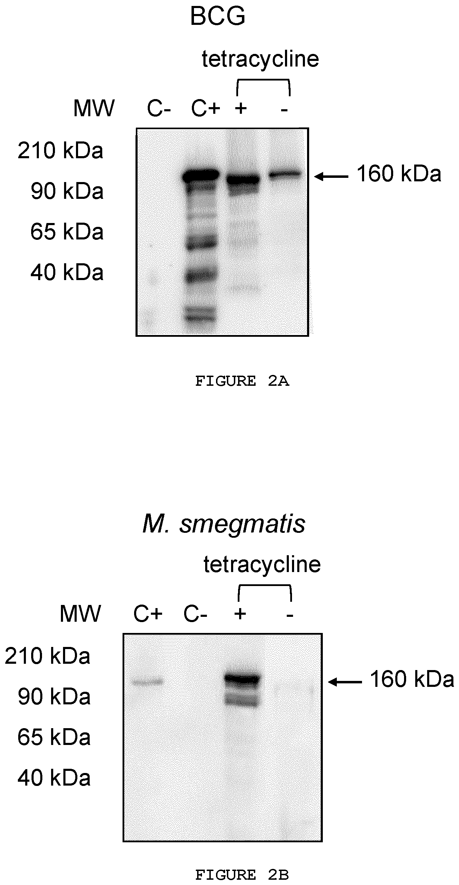

*Cas9* induction was performed for 48h (BCG) and 8h (*M. smegmatis*). Under these conditions, both mycobacterial strains were able to express *Cas9,* as determined by Western blot using an anti-*Cas9* monoclonal antibody (~160 kDa) (Figure 2). Attempts to express wild-type SpCas9 (without codon optimization) in mycobacteria driven by the constitutive P_{GrOEL} promoter were unsuccessful.

Thus, Figure 2 illustrates tetracycline-inducible expression of *Cas9* in BCG and *M. smegmatis.* More specifically, Figure 2 shows the Western blot method of total protein extracts from wild-type (C-) or mycobacterial strains transformed with the pKLM-CRISPR-*lysA* vector (x) induced (+) or not induced (-) with tetracycline. Furthermore, *E. coli* transformed with pCas9 was used as a positive control (C+). Western blots were probed with anti-*Cas9* monoclonal antibody (1:1000). Molecular weight (MW) markers are indicated and the expected molecular weight of *Cas9* indicated by an arrow (~160 kDa). Additionally, Figure 2 shows total protein extracts from wild-type BCG or wild-type *M. smegmatis* (C-); *E. coli* transformed with pCas9 (C+); BCG or *M. smegmatis* induced with tetracycline (+); BCG or *M. smegmatis* not induced (-). The independently isolated clones were analyzed using specific anti-Cas9 monoclonal antibody.

### - Phenotypic screening and characterization of BCGΔlysA and M. smegmatisΔlysA:

After transformation of mycobacteria with pKLM-CRISPR-*lysA*(x), containing the specific sgRNAs to target *lysA* (n=2 for BCG and n=3 sgRNA for *M. smegmatis*) (Table 2), the expression of *Cas9* was induced and isolated colonies recovered from lysine-containing MB7H10 plates. Colonies were transferred to mirror plates with and without lysine to detect functional *lysA* knockout. On BCG, 2/50 of the colonies (4%) were unable to grow without lysine supplementation using crRNA-lysA88-BCG (Figure 3A) and no transformants were obtained using crRNA-lysA20-BCG. In *M. smegmatis,* using crRNA-lysA820-smeg, 2/8 of the colonies (25%) were not able to grow without lysine (Figure 3B). Induction with crRNA-lysA394-smeg did not generate any functional knockout (0/5), while the third did not produce viable transformants. To obtain more inactivations (knockouts), two rounds of induction (independent experiments) were performed on the BCG, obtaining a total of five functional inactivations (knockouts).

Therefore, Figure 3 illustrates a phenotypic screening of BCGΔ*lysA* and *M. smegmatis*Δ*lysA.* After *Cas9* induction, the culture was seeded onto lysine-supplemented plates to recover all viable bacteria. Colonies of A) BCG and B) *M. smegmatis* were then seeded on mirror plates with and without lysine.

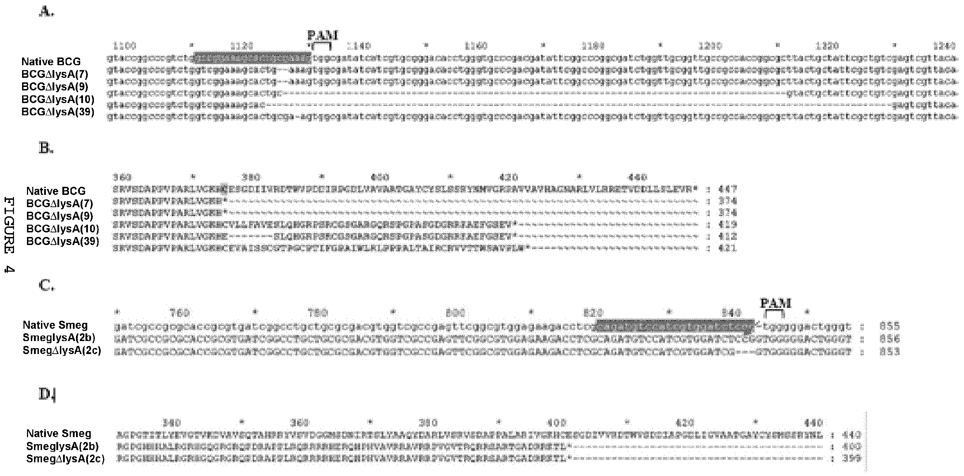

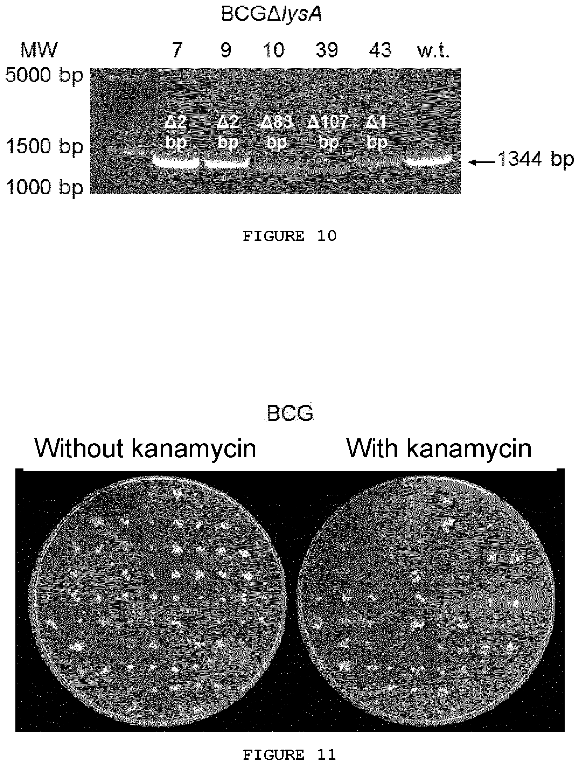

Analysis of the *lysA* region of the respective genomes showed that in BCG, two clones showed the deletion of two nucleotides, one clone had a single nucleotide deleted, while the other two clones showed the removal of 83 and 107 bp (Figure 4A and Figure 10). In *M. smegmatis,* CRISPR/Cas9 induced the removal of two nucleotides in one clone and the addition of a single nucleotide (a cytosine) in another, close to the PAM position (Figure 4B).

In Figure 4, characterization of CRISPR/Cas9-induced *lysA* mutations in A) BCG and B) *M. smegmatis.* The sgRNA used to target the *lysA* gene is highlighted in a gray box, deleted nucleotides are represented by the dashed line, the inserted nucleotide is inside a black box, and the PAM position (NGG) is indicated in square brackets.

### - Evaluation of the potential for reversion to wild-type phenotype:

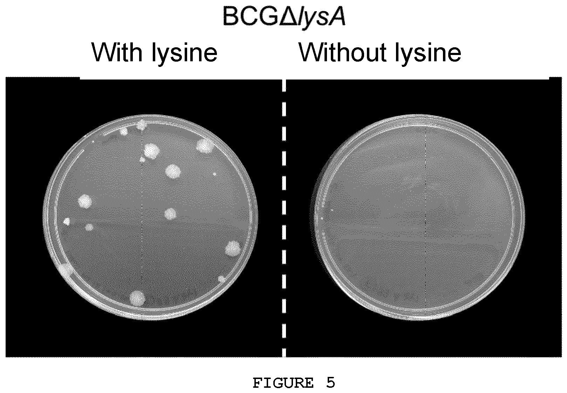

The production of BCG vaccines requires *in vitro* culture for up to 8 passages and is susceptible to mutations in this process. To assess the possibility of reversion to the wild-type phenotype, i.e. the ability to grow without lysine supplementation, the two BCG knockouts showing minimal changes (deletion of only two nucleotides) were used. These clones were grown on MB7H9-OADC plus lysine and on the 8^{th} passage the culture was seeded on MB7H10-OADC with and without lysine. No growth was observed on the plates without lysine supplementation, indicating that reversion to the wild-type phenotype did not occur (Figure 5), a characteristic which is expected for auxotrophic organisms.

Thus, in Figure 5 it is shown that serial passage of BCGΔ*lysA* does not revert to the wild-type phenotype.

### - In vitro growth of complemented auxotrophic mycobacteria:

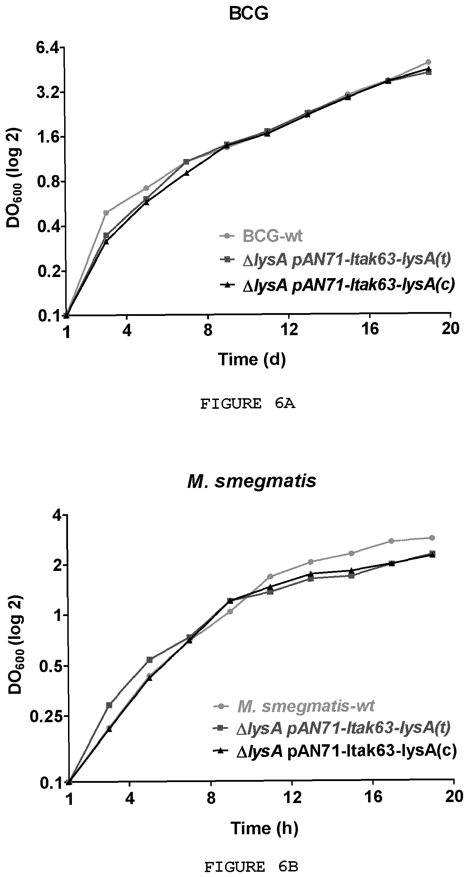

It was investigated whether the complemented auxotrophic strains would show distinct growth rates *in vitro* compared to wild-type mycobacteria. It can be seen that all recombinant *M. smegmatis* and BCG showed growth properties comparable to their wild-type counterparts (Figure 6). Furthermore, no differences were observed between recombinants that drive *lysA* expression in tandem or in cassette with their own promoter.

Therefore, Figure 6 plots the growth curves of complemented auxotrophic strains comparable to wild-type strains. After generating a functional *lysA* knockout, the strains were supplemented with pAN71*-LTAK63-lysA* (t) and pAN71*-LTAK63-lysA* (c) and growth curves compared to wild-type strains. After an initial inoculum at OD₆₀₀ 0.1, regular readings were measured in a spectrophotometer.

### - Expression of LTAK63 adjuvant in recombinant mycobacteria:

Prior to complementation of BCGΔ*lysA* and *M*. *smegmatis*Δ*lysA* with vectors, the mutants underwent a curing process to eliminate the plasmid pKLM-CRISPR-*lysA* (x). Confirmed knockout mutants were inoculated into MB7H9 and incubated at 37°C, 5% CO₂ for 7 days. This inoculum was diluted to a concentration of 10-5 and plated on medium supplemented with lysine. After visible colonies grew, mirror plates of views were made with and without kanamycin. In the first passage, it was observed that 35% of the mutants were susceptible to kanamycin (Figure 11). This feature is very important for vaccine development, as it is important not to have too many passes in the process. Mirror plates were used to select mutants that were not able to grow in the presence of kanamycin. Cured mutants were then transformed with the complementation vectors, pAN71-LTAK63-*lysA* (t) and *pAN71-LTAK63-lysA* (c), and the transformants selected in medium without lysine supplementation.

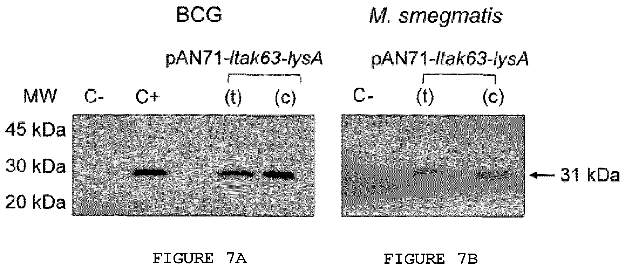

To assess the ability of complemented auxotrophic strains (which express plasmid *lysA*) to express the *LTAK63* antigen, protein extracts from transformants were used to detect *LTAK63* by Western blot. It can be seen that *LTAK63* was produced in both BCG and *M. smegmatis* (Figure 7). The expression of *LTAK63* by the complemented auxotrophic strains was comparable to that observed in BCG transformed with the pAN71-LTAK63 vector, which supports the stability and expression of *LTAK63* through kanamycin.

Thus, Figure 7 represents the expression of *LTAK63* in complemented auxotrophic BCG and *M. smegmatis.* Additionally, the Western blot method of total protein extracts from wild-type BCG or wild-type *M. smegmatis* (C-) is shown; rBCG-*LTAK63* (C+) and auxotrophic strains complemented by tandem (t) or cassette (c) construction. Western blots were probed with mouse antiserum against *LTAK63* (1:1000). Molecular weight (MW) markers are indicated and the expected molecular weight of *LTAK63* indicated by an arrow (~31 kDa).

Therefore, in accordance with the above, the present invention proposes a CRISPR-Cas process for mycobacterial genome modifications through a single vector, preferably for the inactivation of the *lysA* gene using CRISPR/Cas9 to generate a strain of unlabeled auxotrophic BCG.

The results demonstrated the utility of CRISPR/*Cas9* in efficiently mutating at specific positions in the mycobacterial genome. The mutations proved to be irreversible, further reinforcing the use of CRISPR/Cas9 to generate new and improved TB vaccines.

Complementation showed high plasmid stability; together with the expression of *LysA,* these strains were also able to stably express the adjuvant *LTAK63.*

The results suggest that the complemented auxotrophic rBCG strains generated by the present invention would have the necessary properties to induce improved protection against *M. tuberculosis* challenge.

Thus, the embodiments presented in the present invention do not limit the totality of possibilities. It will be understood that various omissions, substitutions and alterations can be made by one skilled in the art, without departing from the spirit and scope of the present invention.

It is expressly provided that all combinations of elements that perform the same function in substantially the same way to reach the same results are within the scope of the invention. Element substitutions from one described embodiment to another are also fully intended and contemplated.

It is also necessary to understand that the drawings are not necessarily to scale, but that they are only conceptual in nature.

Those skilled in the art will appreciate the knowledge presented herein and will be able to reproduce the invention in the embodiments presented and in other variants, covered by the scope of the claims.

### BIBLIOGRAPHIC REFERENCES

Lim, E. M. et al. Identification of Mycobacterium-Tuberculosis DNA Sequences Encoding Exported Proteins by Using PhoA Gene Fusions. Journal of bacteriology 177, 59-65 (1995).

Murray, A. et al. Expression of Escherichia-coli Beta-Galactosidase in Mycobacterium bovis BCG Using an Expression System Isolated from Mycobacterium paratuberculosis Which Induced Humoral and Cellular Immune-Responses. Mol Microbiol 6, 3331-3342, doi:DOI 10.1111/j.1365- 2958.1992.tb02201.x (1992).

NASCIMENTO, I. P.; RODRIGUEZ, D.; SANTOS, C. C.; AMARAL, E. P.; ROFATTO, H. K.; JUNQUEIRA-KIPNIS, A. P.; GONCALVES, E. D. C.; D'IMPERIO-LIMA, M. R.; HIRATA, M. H.; SILVA, C. L.; WINTER, N.; GICQUEL, B.; MILLS, K. H. G.; PIZZA, M.; RAPPUOLI, R.; LEITE, L. C. C. Recombinant BCG Expressing LTAK63 Adjuvant induces Superior Protection against Mycobacterium tuberculosis. Sci Rep, v. 7, n. 1, p. 2109, 2017.

## Claims

1. Process for construction of a single vector comprising *cas9* and sgRNA for mycobacterial genome modifications **characterized in that** it comprises the following steps:
a) Providing the *cas9* expression cassette and the *tracrRNA* expression cassette with codons optimized for expression in mycobacteria and cloning into a cloning vector;
b) Sequentially cloning the sequences optimized in step (a) into a mycobacterial bridge vector consisting of any mycobacterial expression vector comprising a mycobacterial origin of replication and an antibiotic resistance marker;
c) Selecting targeting sequences (crRNAs) specific to the gene of interest;
d) Inserting the crRNAs selected in step (c) into the mycobacterial expression vector, upstream of the *tracrRNA* sequence, at position +1 downstream of an inducible promoter for the construction of the sgRNA cassette; and
e) Adding a transcription terminator at the end of each expression cassette.

2. Process, according to claim 1, **characterized in that** it allows obtaining an "all-in-one" vector for mycobacterial genome modifications such as gene inactivation or deletion (knockout), gene modification or insertion (knockin) or gene silencing (knockdown).

3. Process, according to claim 1 or 2, **characterized in that** the mycobacteria are preferably selected from the group consisting of the tuberculosis complex (*Mycobacterium tuberculosis* (MTB), *Mycobacterium bovis* (BTB), *Bacillus Calmette-Guérin* (BCG) and *Mycobacterium smegmatis*).

4. Process, according to any one of claims 1 to 3, **characterized in that** said mycobacterial bridge vector comprises an antibiotic resistance marker; origin of replication in *E. coli* (oriC) and origin of replication in mycobacteria (oriM), which preferably is the pJH152 vector.

5. Process, according to any one of claims 1 to 4, **characterized in that** to obtain the *cas9* expression cassette, reverse and forward primers complementary to the *cas9* sequence are used in PCR reactions.

6. Process, according to any one of claims 1 to 5, **characterized in that** in step (a) any inducible system is further used, preferably the inducible system *tetR*/tetracycline, wherein in step (a) it is further synthesized the regulatory cassette of the inducible system, preferably the Tet regulatory cassette *(TetR),* wherein reverse and forward primers complementary to the TetR sequence are used in PCR reactions, and preferably from 20 to 2000 ng/mL of tetracycline hydrochloride are used.

7. Process, according to any one of claims 1 to 6, **characterized in that** to obtain the *tracrRNA* expression cassette, reverse and forward primers complementary to the tracrRNA sequence are used in PCR reactions.

8. Process, according to any one of claims 1 to 7, **characterized in that** the optimization in step (a) is carried out by the synthesis of genes with codons optimized for expression in mycobacteria, wherein the optimization of the genes is carried out in a synthetic form, where the nucleotides of the genes to be optimized are modified following the table of codons that are preferentially used by the microorganism to compose the amino acid.

9. Process, according to any one of claims 1 to 8, **characterized in that** in step (b), the cassette of the inducible system used, preferably the *tetR* cassette, is constitutively expressed by pimyc, or another constitutive promoter.

10. Process, according to any one of claims 1 to 9, **characterized in that,** preferably, in step (c) specific crRNAs are selected for the construction of sgRNAs to reach the gene of interest in mycobacteria, wherein the primers complementary reverse and forward to the gene sequence of interest are used in PCR reactions.

11. Process, according to any one of claims 1 to 10, **characterized in that** in step (d) the expression of Cas9 and sgRNA is preferably controlled by a tetracycline-inducible promoter, more preferably a pUV15tetO promoter.

12. Process, according to any one of claims 1 to 11, **characterized in that** additionally in step (e) the restriction sites used for cloning are indicated and two restriction sites are introduced to facilitate the upstream crRNA cloning of *tracrRNA.*

13. Process, according to any one of claims 1 to 12, **characterized in that** it preferably applies the CRISPR/Cas9 system to functionally inactivate (knockout) the LysA expression of mycobacterial strains, wherein the pKLM-CRISPR-lysA(x) vector of SEQ ID NO: 19 is preferably constructed.
